# EUROPEAN PATENT APPLICATION

(11) **EP 2 063 271 A1**
(43) Date of publication of application: **27.05.2009**
(21) Application number: 07807677.5
(22) Date of filing: 13.09.2007
(51) Int. Cl.: G01N 33/574, C12Q 1/34, C12Q 1/37, C12Q 1/527

(54) **TUMOR MARKER FOR RENAL CANCER AND METHOD FOR DETERMINATION OF OCCURRENCE OF RENAL CANCER**

(30) Priority: 15.09.2006 JP 2006251615
(71) Applicant: Shimadzu Corporation, Kyoto-shi Kyoto 604-8511 (JP); National University Corporation Kobe University, Kobe-shi, Hyogo 657-8501 (JP)
(72) Inventor: MASUDA, Taro, Kyoto-shi Kyoto 604-8511 (JP); NISHIMURA, Osamu, Kyoto-shi Kyoto 604-8511 (JP); OKUMURA, Katsuhiko, Kobe-shi Hyogo 657-8501 (JP); OKAMURA, Noboru, Kobe-shi Hyogo 657-8501 (JP); WATANABE, Makoto, Kyoto-shi Kyoto 604-8511 (JP)
(74) Representative: Wilhelms · Kilian & Partner Patentanwälte
(86) International application number: PCT/JP2007/068326
(87) International publication number: WO 2008/032868

(57) **Abstract**

The present invention provides a tumor marker more highly specific to renal cancer. The present invention provides a method for identifying the morbidity of rental cancer. A tumor marker for renal cancer comprising proteins identified from renal cancerous tissue. A method for identifying the morbidity of renal cancer by using the tumor marker for renal cancer. The method comprises measuring the level of the protein in a sample derived from a person of interest who is to be examined to identify the morbidity of renal cancer; and comparing an obtained measured level to a normal level of the protein, wherein an upregulation or a downregulation of the obtained measured level compared to the normal level is used as one of indexes indicating that there is a high possibility that the person of interest has renal cancer.

## Description

### TECHNICAL FIELD

The preset invention relates to a technique for clinical diagnosis, examination, follow-up and classification, more specifically to a tumor marker and a method for identifying the morbidity of cancer disease. Particularly, the present invention relates to a tumor marker for renal cancer and a method for identifying the morbidity of renal cancer, more specifically to a tumor marker for clear cell-type renal cancer and a method for identifying the morbidity or clear cell-type renal cancer.

### BACKGROUND ART

Various tumor makers are being put to practical use for the purpose of early detection of malignant tumors and monitoring of tumors after treatment. Examples or such tumor markers which have already been put to practical use include AFP, BCA225 and the like. These tumor markers are used in clinical practice for diagnosis of hepatocellular cancer and breast cancer, respectively.

In recent years, the importance of biochemical examination of blood, urine, and the like using tumor markers has been further, increasing because such biochemical examination is less stressful on patents. For this reason, research and development on tumor markers are being actively conducted from the viewpoint of both gene and protein. For example, Japanese Patent Application National Publication No. 2004-531713 discloses a tumor marker for use in subtype screening, diagnosis, prognosis, and identification of renal cell cancer.

U.S. Patent No. 5994062 discloses the specific expression of ribonucleoprotein A2 in human lung cancer cell lines (NCI-H720, NCI-157), but does not disclose the relationship of ribonucleoprotein A2 with other tumors.

U. S. Patent Application Publication No. 2004/0029200 discloses a tumor marker for renal cancer in urine.

Japanese Paten Application National Publication No. 2005-530856 reports that heparan sulfate proteoglycan is a gene characteristically expressed in tumor endothelial cellos and normal endothelial cells, but does not disclose that heparan sulfate proteoglycan is a tumor marker protein (or a tumor marker gene) for renal cancer.
Patent Document 1: Japanese Patent Application National Publication No. 2004-531713
Patent Document 2: U. S. Patent No. 5994062
Patent Document 3: U. S. Patent Application Publication No. 2004/0029200
Patent Document 4: Japanese Patent Application National Publication No. 2005-530856

### DISCLOSURE OF THE INVENTION

### Object of the Invention

Renal cancer accounts for 2 to 3% of all malignant tumors, and the number of renal cancer patients is largest among patients with urinary malignant tumors. In general, renal cancer is less likely to cause subjective symptoms and is difficult to detect. In addition, advanced renal cancer has a poor prognosis, and effective therapies for advance renal cancer other than surgical excision have not yet been established. However, a tumor marker useful for diagnosis of renal cancer has not yet been put to practical use, and therefore renal cancer is often accidentally detected by abdominal ultrasound. Under the circumstances, there is a demand in clinical practice for establishment of a method for diagnosing early renal canc.er by biochemical examination. An example of a marker which can be currently used four diagnosis of renal cancer by biochemical examination is BFP. However, this marker has low specificity for cancerous organs, and is therefore used not as a marker specific to renal cancer but as a tumor marker for a wide variety of malignant tumors.

It is therefore an object of the present invention to provides a tumor marker more highly specific to renal cancer. It is also an object of the present invention to provide a method for identifying the morbidity of renal cancer.

### Summary of the Invention

The present inventors have identified, using an NBS method as a proteome analysis method, a group of proteins showing a certain degree or more of difference in their abundance between pathologic tissues and normal tissues sampled from renal cancer patients. The NBS method is an excellent method for relative quantitation of protein/peptide using 2-nittobenzenesulfenyl chloride. A proteome analysis method using the NBS method is fundamentally different from the most versatile proteome analysis method using two-dimensional electrophoresis in separation system and detection principles. Therefore, by using the NBS method as the proteome analysis method, it can be considered that it is passible to find proteins as candidate for markers different from proteins previously reported as candidates for renal cancel markers.

The present invention includes the following.

The following (1) and (2) relate to a tumor marker for renal cancer.

The following (1) relates to a tumor marker for renal cancer including a protein upregulated compared to a normal level in the body of cancer patients.
(1) A tumor marker for renal cancer including at least one protein selected from the group consisting of galectin 1, CNDP dipeptidase 2, reticulocalbin 1, skeletal muscle LIM-protein FHL 1, aldolase A, Enolase I, putative MAPK activating protein (GTRAP3-18), plectin, rigonucleoprotein A2B1, Glucosidase 2 beta subunit., and alpha-crystallin B.
   The renal cancer is preferably clear cell-type renal cancer.
   The following (2) relates to a tumor marker for renal cancer including a protein downregulated compared to a normal lever in the body of cancer patients.
(2) A tumor marker for renal cancer including a protein selected from the group consisting of uromodulin, Aminoacylase 1, aldolase B, heparan sulfate proteoglycan, Aquaportin 1, Lipocalin 7, and Raf kinase inhibitor protein.
   The renal cancer is preferably clear cell-type rental cancer.
   The following (3) to (8) relate to a method for identifying the morbidity of renal cancer. In the present invention, the word "morbidity" widely refers to a diseased state, and the phase "identifying the morbidity of renal cancer" incudes carrying out detection, diagnosis, monitoring, staging, and prognostic evaluation on renal cancer.
   The following (3) to (5) relate to a method for identifying the morbidity of renal canter by using the tumor marker for renal cancer according to the above (1).
(3) A method for identifying the morbidity of renal cancer by using, as a tumor marker for renal cancer, at least one: protein selected from the group consisting of galectin 1, CNDP dipeptidase 2, reticulocalbin 1, skeletal muscle LIM-protein FHL 1, aldolase A, Enolase I, putative MAPK activating protein (GTRAP3-18) plectin, ribonucleoprotein A2B1, Glucosidase 2 beta subunit, and alpha-crystallin B.
(4) The method for identifying the morbidity of renal cancer according to the above (3), comprising measuring the level of the protein in a sample derived from a person of interest who is to be examined to identify the morbidity of renal cancer; and comparing an obtained measured level to a normal level of the protein, wherein an upregulation of the obtained measured level compared to the normal level is used as one of indexes indicating that there is a high possibility that the person of interest has renal cancer.
   The normal level or the protein is any level of the protein, in a sample served as a control for a cancerous sample, and may be a level of the protein in a normal sample derived from a healthy person or a level of the protein in a non-canerous normal sample derived from a renal cancer patient.
(5) The method for identifying the morbidity of renal cancer according to: the above (4), wherein the sample is blond serum or urine and wherein the level of the protein is measured by an examination based on biospecific affinity.
   The renal cancer is preferably clear cell-type renal cancer
   The following (6) to (8) relate to a method for identifying the morbidity of renal cancer by using the tumor marker for renal cancer according to the above (2).
(6) A method for identifying the morbidity of renal cancer by using, as a tumor marker for renal cancer, a protein selected from the group consisting of uromodulin, Aminoacylase 1, aldolase B, heparan sulfate proteoglycan, Aquaportin 1, Lipocalin 7, and Raf kinase inhibitor protein.
(7) The method for identifying the morbidity of renal cancer recording to the above (6), comprising measuring the level of the protein is a sample derived from a person of interest who is to be examined to identify the morbidity of renal cancer; and comparing an obtained measured level to a normal level of the protein, wherein a downlagulation of the obtained measured level compared to the normal level is used at one of indexes indicating that there is a high possibility that the person of interest has renal cancer.
   The normal levee of the protein is any level of the protein in a sample served as a control for a cancerous sample, and may be a level of the protein in a normal sample derived from a healthy person or a level of the protein in a non-cancerous normal sample derived from a renal cancer patient.
(8) The method for identifying the morbidity of renal cancer according to the above (7), wherein the sample is blood serum or urine and wherein the level of the protein is measured by an examination based on biospecific affinity.
   The renal cancer is preferably clear cell-type renal cancer.
   The present invention is also directed to a drug composition for treatment of cancer which will be described in the following (9) and (10). The phrase "treatment of cancer" includes killing of cancer cells and inhibition of the growth of cancer cells.
(9) A drug composition to be supplied to renal cancer cells to induce a reaction promoting killing of renal cancer cells and/or inhibition of the growth of renal cancer cells, said drug composition including at least one antibody to be immunospecifically bound to the tumor marker for renal cancer according to the above (1).
   In particular, the renal cancer is clear cell-type renal cancer.
(10) A drug composition to be supplied to renal cancer cells in an immunostimulating amount to promote an immune response, said drug composition including the tumor marker for renal cancer according to the above (1).
   In particular, the renal cancer is clear cell-type renal cancer.

The drug composition according to the above (9) or (10) can be regarded as a latent therapeutic drug for use in treatment of renal cancer or can be used as therapeutic drug for treatment of renal cancer.

According to the present invention, it is possible to provide a tumor marker more highly specific to renal cancer. In addittion, it is also possible to provide a method for identifying the morbidity of renal cancer.

### BRIEF RESCRIPTION OF THE DRAWINGS

Fig. 1 is (a) an electrophoretogram obtained by western blotting of galectin 1 which is a. candidate protein, (b) the result of quantitative analysis of galectin 1 by western blotting, and (c) the result of quantitative analysis of galectin 1 by an NBS method.
Fig. 2 is (a) an electrophoretogram obtained by western slotting of CNDP dipeptidase 2 which is a. candidate protein, (b) the result of quantitative analysis of CNDP dipeptidase 2. by western blotting, and. (c) the result of quantitative analysis of CNDP dipeptidase 2 by the NBS method.

### MODES FOR CARRYING ODT THE INVENTION

### <Tumor Marker>

The present invention provides a. tumor marker. The tumor marker according to the present invention is used for renal cancer. Further, the tumor marker according to the present invention is particularly useful for clear cell-type renal cancer.

The tumor marker for renal cancer according to the present invention comprises a protein selected from the group consisting of galectin 1, CNDP dipeptidase 2, reticulocalbin 1, skeletal muscle LIM-protein FHL 1, aldolase A, Enolase I (c-myc binding protein), putative MARK activating protein (GTRAP3-18, ADP-ribosylation-like factor 6 interacting protein 5 variant), plectin Heterogeneous nuclear ribonucleoprotein A2B1 (HNRBA2B1 protein), Glucosidase 2 beta subunit (p90, 80K-H=tyrosine-phosphorylated protein), alpha-crystallin B (alpha-crystallin chain B); uromodulin, Aminoacylase 1, aldolase B, heparan sulfate proteoglycan, Aquaportin 1 (uterine water channel, hUMC), Lipocalin 7 (LCN7 protein), and Raf kinase inhibitor protein (neuropolypeptide h3).

These proteins were identified by analyzing renal cancer tissues and normal tissues sampled from patients with clear cell-type renal cancer using a proteome analysis technique including isotopic labeling method (NBS method) using 2-nitrobenzenesulfenyl chloride (NBSCl) and HPLC separation method. The NBS method is a method for determining a relative difference in protein content between two protein samples in different states. More specifically, one of two protein samples in different states is modified with a heavy reagent (2-nitro[¹³C₆]benzenesulfenyl chloride), and the other is modified with a light reagent (2-nitro[¹²C₆]benzenesulfenyl chloride). The thus obtained NBS-modified protein samples are mixed together, and the mixture is subjected to treatment appropriately selected by those skilled in the art, such as tryptic digestion, and a difference in peptide amount is measured by a mass spectrometer. The NBS method is described in, for example, Rapid Common. Mass Spectrom., 2003, 17, 1642-1650 and WO 2004/002950.

A two-dimensional electrophoresis method which .has been widely used has difficulty in separating and detecting a protein with a basic isoelectric point and a protein with a high molecular weight of about 100 KDa. However, the NBS method to be used in the present invention can be carried out without constraints on molecular wight and isoelectric point because peptide fragments obtained by tryptic digestion of proteins contained in tissues are directly analyzed and separation is carried out by HPLC. Therefore, there is a possibility that it is possible to obtain findings different from those previously reported.

These proteins may be isolated and purified by various protein purification techniques selected by those skilled in the art. Examples of these techniques include chromatography techniques such as ion exchange chromatography, affinity chromatography, size exclusion chromatography, and column chromatography, centrifugal separation, difference in solubility, and electrophoresis.

Among the proteins provided as tumor markers for renal cancer according to the present invention, the following proteins are upregulated compared to a normal level in renal cancer patients: galectin 1, CNDP dipeptidase 2, reticulocalbin 1, skeletal muscle LIM-protein FHL 1, aldolase A, Enolase I (c-myc binding protein), putative MAPK activating protein (GTRAP3-18, ADP-ribosylation-like factor 6 interacting protein 5 variant), plectin, Heterogeneous nuclear ribonucleoprotein A2B1 (HNRPR2B1 protein), Glucosidase 2 beta subunit (p90, 80K-H=tyrosine-phosphorylated protein), and alpha-crystallin B (alpha-crystallin chain B).

These proteins tend to be expressed at, for example, about 80% or more, preferably about 100% or more higher level (based on the molar amount) in renal cancer tissues than in normal tissues in about 50% or more of the renal cancer patients.

Among the proteins provided as tumor markers for renal cancer according to the present invention, the following proteins are downregulated compared to a normal level in renal cancer patients: uromodulin, Aminoacylase 1, aldolase B, heparan sulfate proteoglycan, Aquaportin 1 (uterine water channel, hUWC), Lipocalin 7 (LCN7 protein), and Raf kinase inhibitor protein (neuropolypeptide h3).

These proteins tend to be expressed at, for example, an about 80% or more, preferably about 100% or more higher level (bases or the molar amount) in normal tissues than in rental cancer tissues in about 50% or more or the renal cancer patients.

### <Method for Identifying Morbidity or Renal Cancer>

The present invention also provides a method for identifying the morbidity or renal cancer by using, as a tumor marker for renal cancer, at least one protein selected from the group consisting of galectin 1, CNDP dipeptidase 2, reticulocalbin 1, skeletal muscle LIM-proteins FHL 1, aldolase A, Enolase I (c-myc binding protein), putative MAPK activating protein (GTRAP3-18, ADP-ribosylation-like factor 6 interacting protein 5 variant), plectin, Heterogeneous nuclear ribonucleoprotein A2B1 (HNRPA2B1 protein), Glucosidase 2 beta subunit (p90, 80K-H=tyrosine-phosphorylated protein), alpha-crystallin B (alpha-crystallin chain B), uromodulin Aminoacylase 1, aldolase B, heparan sulfate proteoglycan, Aquaportin 1 (uterine water channel, hUWC), Lipocalin 7 (LCN7 protein), and Raf kinase inhibitor protein (neuropolypeptide h3). The method according to the present invention is utilized for identifying the morbidity of renal cancer. Particularly, the method according to the present invention is useful for clear cell-type renal cancer.

According to the method of the present invention, a sample derived from a person of interest who is to be examined to identify the morbidity of renal cancer is prepared, and the level of at least one protein selected from the above-mentioned proteins in the sample is measured. The thus obtained measured level is compared to a normal level. The "normal level" is a level of at least one protein selected from the above-mentioned proteins in a sample served as a control for a cancerous sample. Here, the sample served as a control for a cancerous sample is not particularly limited .a.s long as it is a non-cancerous sample, and may be, for example, a sample derived from a healthy person or a normal sample derived from a renal cancer patient.

In the present invention, the sample: derived from a person of interest who is to be examined to identify the morbidity or renal cancer is not particularly limited, and examples thereof include cells or tissues, body fluids, and tissue extracts. The cells or tissues include tissue biopsy materials and autopsy materials. The body fluids include blood, urine, and body secretions. The blood encompasses whole blood, blood plasma, and blood serum. The tissue extract refers to a tissue homogenated or solubilized by a method well known to those skilled in the art. Among these samples exemplified above, blood serum and/or urine are/is preferred. In addition, the cells or tissues, body fluids, and tissue extracts are preferably derived from the kidney.

The measured level of a tumor marker for renal cancer in a sample containing the cells or tissues, body fluids, and tissue extracts or a person of interest is compared to the level of the tumor marker for rental cancer in a non-cancerous sample containing, preferably, the same kind(s) of cells or tissues, body fluids, and tissue extracts. More specifically, in a case where measured tumor marker for renal cancer is a tumor marker for renal cancer in the blood serum of a person of interest, the measured level is preferably compared to the level of the tumor marker in non-cancerous blood serum.

In a case where the group of proteins is a group consisting of galectin 1, CNDP dipeptidase 2, reticulocalbin 1, skeletal muscle LIM-protein FHL 1, aldolase A, Enolase I (C-myc binding protein), putative MAPK activating protein (GTRAP3-18, ADP-ribosylation-like factor 6 interacting protein 5 variant), plectin, Heterogeneous nuclear ribonucleoprotein A2B1 (HNRPA2BI protein Glucosidase 2 beta subunit (p90, 80K-H=tyrosine-phpsphorylated protein), and alpha-crystallin B (alpha-crystallin chain B), an obtained measured level higher than a normal level may be used as one of indexes indicating that there is a high possibility that a person of interest has renal cancer. An increase degree in the measured level such that the measured level is 80% or more, preferably 100% or more higher than the normal level (based on the molar amount) may be a criterion.

In a. case where the group of proteins is a group consisting of uromodulin, Aminoacylase 1, aldolase B, heparan sulfate proteoglycan, Aquaportin 1 (uterine water channel, hUWC), Lipocalin 7 (LCN7 protein), and Raf kinase inhibitor protein (neuropolypeptide h3), an obtained measured level lower than a normal level may be used as one of indexes indicating that there is a high possibility that a person of interest has renal cancer. A decrease degree in the measured level such that the normal level is 80% or more, preferably 100% or more higher than the measured level (based on the molar amount) may be a criterion.

The level of the protein is preferably measured by an examination based on biospecific affinity. Such an examination based an biospecific affinity is well known to those skilled in the art, and is not particularly limited. However, an immunoassay is preferred. Specific examples of the immunoassay include immunoassay comprising competitive and noncompetitive assay systems such as western blotting, radioimmunoassay, ELISA, sandwich immunoassay, immunoprecipitation, precipitation reaction, gel diffusion precipitation reaction, immunodiffusion, aggregation measurement, complement fixation assay, inmunoradiometric assay, fluorescence immunoassay, and protein A immunoassay. The immunoassay is carried out to detect the presence of an antibody bound to a tumor marker in the sample of an individual. More specifically, such an immunoassay is carried out by bringing the sample into contact with an antibody against a tumor marker protein of interest in an assay medium under conditions where the tumor marker protein and the antibody can form an immune complex. A more specific immunoassay protocol may be easily selected by those skilled in the art.

As described above, the level or the protein is preferably measured by an examination based on biospecific affinity, but may be measured also by the other protein quantitation methods. For example, the NBS method described above is an excellent quantitation method. In this case, the level of the protein may be measured by determining a difference in the existing amount of the protein between the sample or a person or interest and an appropriate control sample such as a sample prepared so as to contain a known level of the protein for a normal sample.

According to the method of the present invention, the tumor marker for renal cancer according to the present invention may be measured alone or in combination with any other tumor marker. Therefore, the method according to the present invention may include measuring the level of the other tumour markers in addition to measuring the level of the tumor marker for renal canter according to the present invention.

The tumor marker for renal cancer according to the present invention may be used for detection, diagnosis, monitoring, staging, and prognostic evaluation of renal cancer, and is preferably used to grasp tumor kinetics. For example, in the case of treatment of a tumor by chemotherapy or radiotherapy, the tumour marker may be used to determine how much effect the therapy has had. Further, in the case of treatment of a tumor showing a high tumor marker level by excisional surgery, the tumor marker may be used for postoperative follow-up.

### <Drug Composition>

The present invention is also directed to a drug composition for treatment of renal cancer by using the tumor marker containing a protein unregulated compared to a normal level in renal cancer patients of tumor marker for renal cancer according to the present invention.

One embodiment of the present invention provides a drug composition to be supplied to renal cancer cells to induce a reaction promoting killing of renal cancer cells and/or inhibition or the growth of renal cancer cells, the drug composition including at least one antibody to be immunospecifically bound to the tumor marker for renal cancer according to the present invention. The term antibody encompasses polyclonal antibodies, monoclonal antibodies, and antibodies prepared by molecular biologic techniques. Here, the antibody is not particularly limited as long has it is broadly a material which can be immunospecifically bound. For example, antibody fragments and antibody fusion proteins may also be used. In each case, such an antibody is prepared by a method well known to those skilled in the art.

Another embodiment of the present invention provides a drug composition to be supplied to renal cancer cells in an immunostimulating amount to promote an immunoresponse, the drug composition including the tumor marker for renal cancer according to the present invention. Here, the immunostimulating amount refers to the amount of an antigen capable of inducing a desired immunoreaction for treatment of cancer, and is determined by a method well know to those skilled in the art. By using such a drug composition, it is possible to carry out treatment of cancer, known as the so-called cancer vaccine therapy, by a method well known to those skilled in the art.

The drug composition according to the present invention includes, as an active ingredient, the above-described antibody or tumor marker, but may further include a pharmaceutically acceptable diluent, carrier, excipient, of the like. The drug composition according to the present invention may be regarded as a latent therapeutic drug for use in treatment of renal cancer or may be used as a therapeutic drug for treatment of renal cancer.

### EXAMPLES

The present invention will be more specifically described with reference to the following example, but is not limited to this example. It is to be noted that the amount represented by "%" is based on volume unless otherwise specified.

### <Example 1>

### (1) Tissue extirpated from renal cancer patients

14 kinds of renal cancer tissues, that was extirpated from 14 patients diagnosed as renal cancer and from whom informed consent had been obtained, and was confirmed the observation as clear cell carcinoma according to WHO claissification, were used as samples. The age, sex, and stage of cancer progression (at the time of tissue extirpating) of the patients are shown in Table 1.

**Table 1**

| patient No. | sex | staging | tissue type |
|---|---|---|---|
| No. 3 | M | pT3a | clear |
| No.10 | F | pT3b | clear |
| No.13 | F | pT3a | clear |
| No.28 | F | pT3a | clear |
| No.31 | M | pT3b | clear |
| No.33 | M | pT3a | clear |
| No.35 | M | pT3b | clear |
| No.36 | M | pT3a | clear |
| No.37 | M | pT3a | clear |
| No.39 | F | pT3b | clear |
| No.41 | F | pT3b | clear |
| No.42 | F | pT3b | clear |
| No.43 | M | pT3a | clear |
| No.44 | F | pT3b | clear |

On the other hand, renal normal tissues extirpated from the same patients (hereinafter, simply referred to as "normal tissues") were used as control samples.

The samples to be used for the experiment were immediately cut after extirpation, frozen in liquid nitrogen, and then immediately transferred into a freezer (-80°C) and stored until use.

### (2) Protein extraction from tissue sample

The cancerous tissue and the normal tissue sampled from the same patients were each treated in the following manner to obtain a "soluble fraction" and an "insoluble fraction".

20 mg of' the tissue was placed in a tube, and 200 µL of a "soluble protein extracting reagent" (which will be described later) and 5 zirconia beads were added to the tube to crush the tissue for 5 minuter by a crusher. A rotor including the sample was cooped, and then the tissue was again crushed in the same manner. The thus obtained tissue debris suspension was transferred into another tube and centrifuged (20,000 ×g, 15 minutes) to separate the suspension into a supernatant faction and a precipitate fraction. The supernatant fraction was directly transferred into another tube to obtain a "soluble fraction". On the other hand, the precipitate fraction was washed with the "soluble protein extracting reagent" three times, and then 200 µL of an "insoluble protein solubilizing reagent" (which will be described later) was added whereto for solubilization to obtain an "insoluble fraction".

### <Soluble Protein Extracting Reagent>

50 mM Tris-HCl pH = 8.8
0,1 M NaCl
100 mM PMSF
1/100 volume protease inhibitor cocktail (SIGMA) was added to the above.

6M Urea
2M Thiourea
3% CHAPS
1% TritonX-100
1/100 volume protease inhibitor cocktail (SIGMA) was added to the above.

100 µL of each of the "soluble fraction" and the "insoluble fraction" obtained from a cancerous tissue and the "soluble fraction" and the "insoluble fraction" obtained from a normal tissue was treated with 2D-clean up kit (Bio-Rad) to precipitate protein components. The thus: obtained precipitate was added and completely dissolved in 50 µL of a dissolving solution 1 (5 mM EDTA, 6 M guanidine hydrochloric acid) of ¹³C NBS Stable Isotope Labeling Kit-N (Shimadzu Corporation). The protein concentration of each of the thus obtained samples was measured by BCA Protein Assay (Pierce), and was adjusted to 100 µg/25 µL.

### (3) Protein labeling, tryptic digestion, and concentration of labeled peptides

Among these samples whose protein concentration was adjusted, the sample derived from the soluble fraction of a normal tissue and the sample derived from the insoluble fraction of a normal tissue were labeled with ¹²C NBS (Light NBS), and the sample derived from the soluble fraction of a cancerous tissue and the sample derived from the insoluble fraction of a cancerous tissue were labeled with ¹³C NBS (Heavy NBS) to obtain labelled protein samples. Labeling with Light NBS or Heavy NBS was carried out according to the recommended protocol supplied with ¹³C NBS Stable Isotope Labeling Kit-N (Shimadzu Corporation). Then, the labeled protein sample derived from the soluble fraction of a normal tissue and the labeled protein sample derived from the soluble fraction of a cancerous tissue were mixed together to prepare a soluble fraction-derived protein mixed sample. On the other hand, the labelled protein sample derived from the insoluble fraction of a normal tissue and the labeled protein sample derived from the insoluble fraction of a cancerous tissue were mixed together to prepare an insoluble fraction-derived protein mixed sample.

Then, the soluble fraction-derived protein mixed sample and the insoluble fraction-derived protein mixed sample were each subjected to the steps of deionization, reduction, alkylation, and tryptic digestion. These steps were also carried out according to the recommended protocol supplied with the kit. In this way, a soluble fraction-derived digest sample was obtained from the soluble fraction-derived protein mixed sample, and an insoluble fraction-derived digest sample was obtained from the insoluble fraction-derived protein mixed sample.

Then, the soluble fraction-derived digest sample and the insoluble fraction-derived digest sample were each subjected to the step of concentrating labelled peptides. This step was carried out by a stepwise method described in the recommenced protocol supplied with the kit. In this way, a 10% acetonitrile fraction (fr1), a 15% acetonitrile traction (fr2), a 20% acetonitrile fraction (fr3), a 25% acetanitrile fraction (fr4), a 30% acetonitrile fraction (fr5), a 35% acetonitrile fraction (fr6), a 40% acetonitrile fraction (fr7), and a 50% acetonitrile fraction (fr8) were obtained from each of the samples.

### (4) Reparation of labeled peptides by HPLC

Each of the fractions obtained through concentration carried out by the stepwise method was dried using a centrifugal condensing dryer. The thus obtained dry solids were each added and dissolved in 10 µL of a 10% aqueous acetonitnie solution containing 0,1% TFA to prepare solution of fr1 to fr8. Then the solutions of fr1 and fr 2 wee mixed together, the solutions of fr3 and fr4 were mixed together, the solutions of fr5 and fr6 were mixed together, and the solutions of fr7 and fr8 were mixed together to prepare 4 kinds of solutions.

The obtained 4 kinds of solutions were subjected to separation by HPLC. The initial conditions for HPLC were acetonitrile 14%; 0.1% TFA; and flow rate 1.0 µL/min. The HPLC was carried out until the gradient conditions for peptide elution were 2.9%/min; 20 minutes; and final acetonitrile concentration 72%. Peptides eluted by HPLC were fractionated onto a 384-well sample plate for Axima by using AccuSpot (Shimadzu Corporation). The fractionation start times were as follows: fr1 solution + fr2 solution, 7 minutes later; fr3 solution + fr4 solution 7.5 minutes later; fr5 solution + fr6 solution, 8 minutes later; and fr7 solution + fr8 solution, 9 minutes later. A matrix solution to be used was prepared so that the final concentrations of CHCA (α-cyanohydroxycinnamic acid) and HNBA (hydroxynitrobenzoic acid) were each 10 mg/mL.

### (5) Mass spectrometry and screening of candidate peptides

The fractionated samples were spotted onto a sample plate to carry out mass spectrometry using Axima-CFR plus (Shimadzu Corporation) in reflectron mode. Mass calibration was carried out by an external standard method using ACTH fragment 18-39 (Sigma) and Bradykinin fragment 1-7 (Sigma) as standard samples. From the thus obtained mass spectra, monoisotopic peaks were selected using Mascot Distiller (Matrix science), and paired peaks of NBS-labeled peptides having a mass difference of 6 Da or 12 Da were extracted by software "NBS analysis" (Shimadzu Corporation). Among a group of the paired peaks obtained from the samples, those showing a difference in peak intensity between a normal tissue and a cancerous tissue were picked up.

From the picked-up paired peaks, marker candidates were selected in the following manner. First, peptides whose amount in a cancerous tissue tended to increase or decrease compared to a normal tissue in 7 or more of the 14 patients were basically selected. The criterion of degree of increase or decrease is se as follows. Namely, in the case of peptides whose amount in a cancerous tissue was higher than in a normal tissue, those whose amount in a cancerous tissue was 80% or more higher than in a normal tissue were selected. On the other hand, in the case of peptides whose amount in a cancerous tissue was lower than in a normal tissue, those whose amount in a normal tissue was 80% or more higher than in a cancerous tissue were selected. It is to be noted that the abundance represented by % is based on the peak area.

### (6) MS/MS analysis of proteins as marker candidates and protein identification by database search

Among the peaks showing such a difference in expression level as described above, 62 peptides showed upregulation in a cancerous tissue and 163 peptides showed downregulation in a cancerous tissue. Among the 62 peptides which trended to be upregulated in a cancerous tissue, 39 peptides were identified is the following manner. Among the 163 peptides which tended to be downregulated in a cancerous tissue, 76 peptides were identified in the following manner.

These peptides selected as marker candidates were analyzed by MS/MS ion search using Axima-QIT (Shimadzu Corporation). The thus obtained mass spectra were processed using Mascot Distiller (Matrix science) to carry out protein identification by Mascot search (Matrix science). Proteins with a score obtained from MS/MS ion search by Mascot search of 20 or higher were selected as marker candidates.

Tables 2 to 13 show the result of quantitative analysis of a group of peptides, whose amount tended to be higher in a cancerous tissue than in a normal tissue, in both normal and cancerous tissues and the result of protein identification. In each of Tables 2 to 13, the data number and name of the identified protein shown in the NCBI Home Page, the patient numbed corresponding to that shown in Table 1, the mass/charge values of paired peaks corresponding to the labeled peptides detected by mass spectrometry and having a mass difference of 6 Da or 12 Da, and the ratio between the amount of the protein in both tissues of each of the patients calculated based on the peak areas of the labeled peptides are shown. The ratio between the amount of the protein in both tissues of each of the patients was determined by defining a smaller one of the amount of the protein in a normal tissue and that in a cancerous tissue as 100. Further, in Tables 2 to 13, only in cancerous tissue" means that the protein was not detected in a normal tissue but detected in a cancerous tissue, and "not detected" means that the proteins was detected in neither a normal tissue nor a cancerous tissue.

It is to be noted that the proteins claimed in claim 1 are shown in Tables 2 to 9 and Tables 11 to 13,

**Table 2**

| gi\|252093 (galectin1) | | |
|---|---|---|
| patient No. | m/z 1229.1 normal tissue | m/z 1235.5 cancerous tissue |
| No. 3 | 100. | 320.5 |
| No.10 | 100 | 511.9 |
| No.13 | 100 | 373.6 |
| No.28 | not detected | |
| No.31 | 100 | 242.4 |
| No.33 | 100 | 197.7 |
| No.35 | 100 | 210.6 |
| No.36 | 100 | 350.9 |
| No.37 | 100 | 272.6 |
| No.39 | 235 | 100 |
| No.41 | 100 | 364.8 |
| No.42 | 231.7 | 100 |
| No.43 | 100 | 209.1 |
| No.44 | 100 | 498.3 |

**Table 3**

| gi\|13112005 (CNDP dipeptidase 2:) | | |
|---|---|---|
| patient No. | m/z 1963 normal tissus | m/z 1975 cancerous tissue |
| No. 3 | not detected | |
| No.10 | 100 | 454.4 |
| No.13 | 100 | 428.8 |
| No.28 | not detected | |
| No.31 | 100 | 446.1 |
| No.33 | 100 | 369 |
| No.35 | 100 | 562.9 |
| No.36 | 100 | 415 |
| No.37 | 100 | 182.3 |
| No.39 | not detected | |
| No.41 | not detected | |
| No.42 | 462.8 | 100 |
| No.43 | 100 | 788.2 |
| No.44 | 100 | 253.1 |

**Table 4**

| gi\|14603330 (reticulocalbin precursor) | | |
|---|---|---|
| patient No. | m/z 2102.8 normal tissue | m/z 2108.8 cancerous tissue |
| No. 3 | not detected | |
| No.10 | 100 | 388.1 |
| No.13 | 100 | 774.4 |
| No.28 | not detected | |
| No.31 | 100 | 384.5 |
| No.33 | 100 | 275.6 |
| No.35 | 100 | 540.6 |
| No.36 | 100 | 399.1 |
| No.37 | 100 | 329 |
| No.39 | not detected | |
| No.41 | not detected | |
| No.42 | 210.9 | 100 |
| No.43 | 100 | 544.6 |
| No.44 | 100 | 680 |

**Table 5**

| gi\|2853224 (skeletal muscle LIM-protein FHL1) | | |
|---|---|---|
| patient No. | m/z 1321.5 normal tissue | m/z 1327.5 cancerous tissue |
| No. 3 | 100 | 2542 |
| No.10 | not detected | |
| No.13 | not detected | |
| No.28 | 100 | 118.6 |
| No.31 | 100 | 130.9 |
| No.33 | 100 | 436.8 |
| No.35 | 100 | 261.7 |
| No.36 | 100 | 652.5 |
| No.37 | 100 | 218.1 |
| No.39 | | only in cancerous tissue |
| No.41 | not detected | |
| No.42 | 100 | 299 |
| No.43 | | only in cancerous tissue |
| No.44 | 100 | 277.6 |

**Table 6**

| gi\|28614 (aldolase A) | | |
|---|---|---|
| patient. No. | m/z 1962 normal tissue | m/z 1968 cancerous tissue |
| No. 3 | 100 | 228 |
| No. 10 | not detected | |
| No.13 | 100 | 346.6 |
| No.26 | not detected | |
| No.31 | 100 | 365.3 |
| No.33 | 100 | 886 |
| No.35 | 100 | 693.1 |
| No.36 | | only in cancerous tissue |
| No.37 | 100 | 868 |
| No.39 | not detected | |
| No.41 | 100 | 363.2 |
| No.42 | 336.5 | 100 |
| No.43 | 100 | 377.1 |
| No.44 | 100 | 865 |

**Table 7**

| gi\|1180663 (c-mycbindingprotein (EnolaseI)) | | |
|---|---|---|
| patient No. | m/z 1794.8 normal tissue | m/z 1700.8 cancerous tissue |
| No. 3 | 100 | 213.9 |
| No.10 | 10.0 | 477.7 |
| No.13 | 100 | 1566.9 |
| No.28 | 278.1 | 100 |
| No.31 | 100 | 266.7 |
| No.33 | 100 | 396.8 |
| No.35 | 100 | 1001.5 |
| No.36 | 100 | 944.9 |
| No.37 | 100 | 229.4 |
| No.39 | 203.9 | 100 |
| No.41 | 135 | 100 |
| No.42 | 747 | 100 |
| No.48 | 120 | 100 |
| No.44 | 100 | 506.7 |

**Table 8**

| gi\|183150718 (MAP kinase activating protein (GTRAP3-18)) | | |
|---|---|---|
| patient No. | m/z 1465.6 normal tissue | m/z 1471.6 cancerous tissue |
| No. 3 | not detected | |
| No.10 | 100 | 385.7 |
| No.13 | not detected | |
| No.28 | 100 | 337.1 |
| No.31 | 184.3 | 100 |
| No.33 | 100 | 352.1 |
| No.35 | 100 | 313.6 |
| No.36 | 100 | 284.7 |
| No.37 | 100 | 179 |
| No.39 | 100 | 985 |
| No.41 | not detected | |
| No. 42 | not detected | |
| No.43 | 100 | 396 |
| No.44 | 100 | 194 |

**Table 9**

| gi\| 1296662 (plectin) | | |
|---|---|---|
| patient No. | m/z 1682.9 normal tissue | m/z 1688.9 cancerous tissue |
| No. 3 | not detected | |
| No.10 | 100 | 262.8 |
| No.13 | 100 | |
| No.28 | not detected | |
| No.31 | 100 | 115.8 |
| No.33 | 100 | 565.2 |
| No.35 | 100 | 250 |
| No.36 | 100 | 376.7 |
| No.37 | 100 | 141 |
| No.39 | not detected | |
| No.41 | 100 | 222.6 |
| No.42 | not detected | |
| No.43 | 100 | 203.9 |
| No.44 | 100 | 204.9 |

**Table 10**

| gi\| 31397 (fibronectin precursor) | | |
|---|---|---|
| patient No. | m/z 2080.1 normal tissue | m/z 2086.1 cancerous tissue |
| No.3 | 100 | 640 |
| No.10 | 100 | 223.2 |
| No.13 | not detected | |
| No. 28 | not detected | |
| No.31 | 100 | 666.7 |
| No.33 | 100 | 605.7 |
| No.35 | 318.7 | 100 |
| No.36 | 100 | 146 |
| No.37 | 100 | 107 |
| No.39 | not detected | |
| No.41 | 100 | 2311 |
| No.42 | 100 | 757.7 |
| No.43 | 100 | 481.1 |
| No.44 | 100 | 190.4 |

**Table 11**

| gi\| 500638 (Heterogeneous nuclear ribonucleoprotein A2B1) | | |
|---|---|---|
| patient No. | m/z 1240.6 normal tissue | m/z 1246.6 cancerous tissue |
| No.3 | not detected | |
| No.10 | 100 | 613.8 |
| No.13 | 100 | 109.6 |
| No.28 | 145 | 100. |
| No.31 | 100 | 330.8 |
| No.33 | 100 | 616.5 |
| No.35 | 100: | 1023 |
| No.36 | 100 | 329.4 |
| No.37 | 100 | 237.5 |
| No.39 | 100 | 237.4 |
| No.41 | 153 | 100 |
| No.42 | 775.6 | 100 |
| No.43 | 100 | 202.7 |
| No.44 | 100 | 453.8 |

**Table 12**

| gi\| 1438753 (p90, 80K-H=tyrosine-phosphorylated protein) | | |
|---|---|---|
| patient No. | m/z 1705.9 normal tissue | m/z 1711.09 cancerous tissue |
| No. 3 | 108.1 | 100 |
| No.10 | 100 | 178.6 |
| No.13 | 100 | 32.6.1 |
| No.28 | 163 | 100 |
| No.31 | 100 | 135.9 |
| No.33 | 100 | 196.7 |
| No.35 | 100 | 38.7 |
| No.36 | 100 | 195 |
| No.37 | 100 | 359.9 |
| No.39 | 424.3 | 100 |
| No.41 | 486.4 | 100 |
| No.42 | not detected | |
| No.43 | 100 | 267.8 |
| No.44 | 100 | 200.2 |

**Table 13**

| gi\| 2852648 (crystallin, alpha B) | | |
|---|---|---|
| patient No. | m/z 1649.8 normal tissue | m/z 1655.8 cancerous tissue |
| No.3 | 100 | 334.6 |
| No.10 | 100 | 461.8 |
| No.13 | not detected | |
| No.28 | 100 | 935.5 |
| No.31 | 100 | 437.7 |
| No.33 | 100 | 335.7 |
| No.35 | 100 | 366.9 |
| No.36 | 100 | 373.2 |
| No.37 | 100 | 275.7 |
| No.39 | 1000 | 265.2 |
| No.41 | 100 | 266.6 |
| No.42 | 100 | 200 |
| No.43 | 376.8 | 100 |
| No.44 | 100 | 581.6 |

Tables 14 to 20 show the result of quantitative analysts of a group of peptides, whose amount tended to be higher in a normal tissue than in a cancerous tissue in both normal and cancerous tissues and the result of protein identification as in the caste of Tables 2 to 13. In Tables 14 to 20, "only in normal tissue" means that the protein was not detected in a cancerous tissue but detected in a normal tissue.

It is to be noted that the proteins claimed in claim 3 are shown in Tables 14 two 20.

**Table 14**

| gi\| 340166 (uromodulin) | | |
|---|---|---|
| patient No. | m/z 1282.6 normal tissue | m/z 1288.6 cancerous tissue |
| No.3 | not detected | |
| No.10 | only in normal tissue | |
| No.13 | only in normal tissue | |
| No.28 | only in normal tissue | |
| No.31 | only in normal tissue | |
| No.33 | not detected | |
| No.35 | only in normal tissue | |
| No.36 | only in normal tissue | |
| No.37 | only in normal tissue | |
| No.39 | not detected | |
| No.41 | only in normal tissue | |
| No.42 | only in normal tissue | |
| No.43 | only in normal tissue | |
| No.44 | only in normal tissue | |
| No.44 | only in normal tissue | |

**Table 15**

| gi\| 15559494 (Aminoacylase 1) | | |
|---|---|---|
| patient No. | m/z 1136.5 normal tissue | m/z 1148.5 cancerous tissue |
| No.3 | only in normal tissue | |
| No. 10 | only in normal tissue | |
| No.13 | not detected | |
| No.28 | not detected | |
| No.31 | not detected | |
| No.33 | only in normal tissue | |
| No.35 | not detected | |
| No.36 | only in nomal tissue | |
| No.37 | only in normal tissue | |
| No. 39 | only in normal tissue | |
| No.41 | only in normal tissue | |
| No.42 | only in normal tissue | |
| No.43 | 1114.6 | 100 |
| No.44 | only in normal tissue | |

**Table 16**

| gi\| 178357 (aldolase B) | | |
|---|---|---|
| patient No. | m/z 1402.7 normal tissue | m/z 1408.7 cancerous tissue |
| No. 3 | only in normal tissue | |
| No.10 | only in normal tissue | |
| No.13 | only in normal tissue | |
| No.28 | not detected | |
| No.31 | not detected | |
| No.33 | only in normal tissue | |
| No.35 | only in normal tissue | |
| No.36 | only in normal tissue | |
| No.37 | onyl in normal tissue | |
| No.39 | only in normal tissue | |
| No.41 | only in normal tissue | |
| No.42 | only in normal tissue | |
| No.43 | not detected | |
| No.44 | only in normal tissue | |

**Table 17**

| gi\| 184427 (heparan sulfate proteoglycan) | | | | | | |
|---|---|---|---|---|---|---|
| patient No. | m/z 1675.9 normal tissue | m/z 1681.9 cancerous tissue | m/z 1347.03 normal tissue | m/z 1353.07 cancerous tissue | m/z 2024.49 normal tissue | m/z 2030.5 cancerous tissues |
| No. 3 | 254.4 | 100 | 365 | 100 | 263 | 100 |
| No.10 | 232.9 | 100 | not detected | | not detected | |
| No.13 | 508.3 | 100 | 398 | 100 | not detected | |
| No.28 | 6163.5 | 100 | not detected | | not detected | |
| No.31 | 467.6 | 100 | 565.3 | 100 | not detected | |
| No.33 | 675.6 | 100 | 237.3 | 100 | not detected | |
| No.35 | 343.8 | 100 | 351.6 | 100 | 301.8 | 100 |
| No.36 | 930.8 | 100 | 1052.7 | 100 | 580.6 | 100 |
| No.37 | 548.2 | 100 | only in normal tissue | | 2775.2 | 100 |
| No.39 | 2202.6 | 100 | only in normal tissue | | 4271.4 | 100 |
| No.41 | only in normal tissue | | 514.1 | 100 | 212.9 | 100 |
| No.42 | only in normal tissue | | not detected | | not detected | |
| No.43 | 306.3 | 100 | 369.1 | 100 | 251.1 | 100 |
| No.44 | 948.3 | 100 | 1781.8 | 100 | 968.1 | 100 |

**Table 18**

| gi\|913159(neuropolypeptide h3 (Raf kinase inhibitor)) | | | | |
|---|---|---|---|---|
| patient No. | m/z 1786 normal tissue | m/z 1792 cancerous tissue | m/z 1862.8 normal tissue | m/z 1868.84 cancerus tissue |
| No.3 | 226.7 | 100 | 305.6 | 100 |
| No.10 | 473.4 | 100 | not detected | |
| No.13 | not detected | | not detected | |
| No.28 | 2378.7 | 100 | 100 | 233.8 |
| No.31 | 311 | 100 | 351.6 | 100 |
| No.33 | 256.1 | 100 | 294 | 100 |
| No.35 | not detected | | not detected | |
| No.36 | 318.8 | 100 | 322.2 | 100 |
| No.37 | 410.3 | 100 | 481.2 | 100 |
| No.39 | 2625.8 | 100 | only in normal tissue | |
| No.41 | 3375.5 | 100 | only in normal tissue | |
| No.42 | only in normal tissue | | only in normal tissue | |
| No.43 | 255 | 100 | 1328.8 | 100 |
| No.44 | 299.7 | 100 | 214.6 | 100 |

**Table 19.**

| gi\|688358 (uterine water channel; hUWC (Aquaportin 1)) | | |
|---|---|---|
| patient No. | m/z 2465.1 Normal tissue | m/z 2471.1 cancerous tissue |
| No. 3 | only in normal tissue | |
| No.10 | 329.3 | 100 |
| No.13 | not detected | |
| No.28 | not detected | |
| No.31 | only in normal tissue | |
| No.33 | 1043 | 100 |
| No.35 | 557.4 | 100 |
| No.36 | 1277.9 | 100 |
| No.37 | only in normal tissue | |
| No.39 | only in normal tissue | |
| No.41 | only in normal tissue | |
| No.42 | not detected | |
| No.43 | not detected | |
| No.44 | only in normal tissue | |

**Table 20**

| gi\|14290553 (LCN7 protein (Lopocalin 7) | | |
|---|---|---|
| patient No. | m/z 1583.8 Normal tissue | m/z 1589.8 cancerous tissue |
| No. 3 | 208.2 | 100 |
| No.10 | 204.4 | 100 |
| No.13 | 232.5 | 100 |
| No.28 | only in normal tissue | |
| No.31 | 239.3 | 100 |
| No.33 | 441 | 100 |
| No.35 | 335.2 | 100 |
| No.36 | only in normal tissue | |
| No.37 | only in normal tissue | |
| No.39 | not detected | |
| No.41 | 662.8 | 100 |
| No.42 | 507 | 100 |
| No.43 | only in normal tissue | |
| No.44 | 651.6 | 100 |

### (7) Validation by western blotting

Western blotting was carried out using antibodies specific to the identified proteins as marker candidates in the following manner to validate that these proteins were present in the tissues of the patients. Here, among the candidate proteins showing an upregulation in a cancerous tissue compared to a normal tissue in the NBS method described in the above (5), galectin 1, CNDP dipeptidase 2, reticulocalbin 1, aldolase A, Enolase I, Glucosidase 2 beta subunit, and alpha-crystallin chain B were subjected to western blotting.

First, a soluble fraction (one described in the above (2)) extracted from each of the patients was developed by 12.5% SDS-PAGE, and was then transferred onto a PVDF membrane by semi-dry blotting. The PVDF membrane was blocked with 20 mM TBS containing 5 wt% skim milk and treated with primary antibodies against these proteins as marker candidates such as rabbit anti-human galectin 1 and anti CNDP2 for 30 minutes. Operations after treatment with secondary antibodies were curried out using Elite ABC Kit peroxidase (Rabbit IgG) (Vector Laboratories) according to a manufacturer's recommended method. As a substrate for peroxidase and a color reaction reagent, Immunostain HRP (Konica-Minolta) was used.

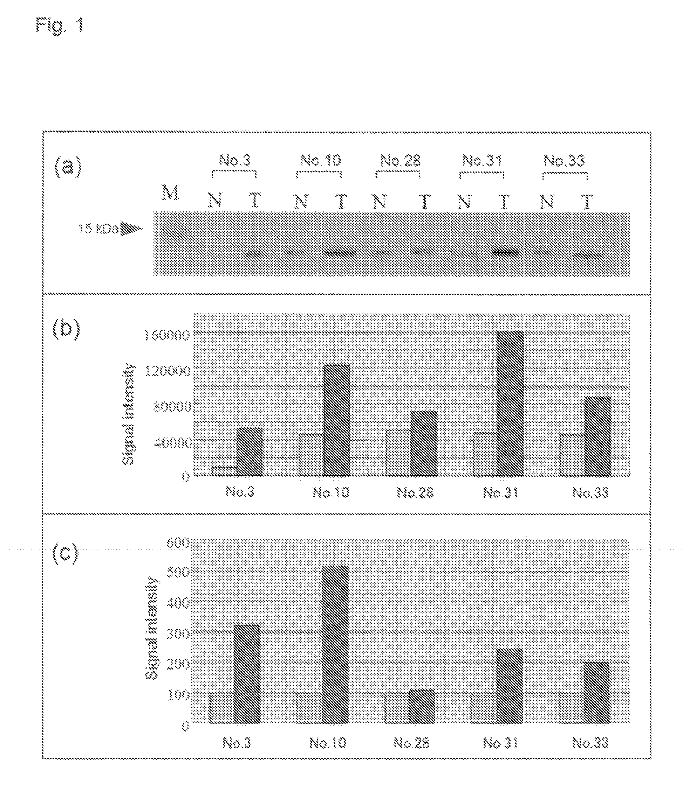

Fig. 1 shows the results of analysis of galectin 1 as a candidate protein.
Fig. 1(a) is an electrophoretogram obtained by western blotting. In Fig. 1(a), M represents a molecular weight marker, N represents a normal tissue-derived sample, and T represents a cancerous tissue-derived sample, and the number (No.) corresponds to the patient number shown in Table 1 (the same goes for Figs. 1(b) and 1(c)).
Fig. 1(b) shows the result of a comparison of the quantity of the protein determined by digitizing a signal detected in Fig. 1(a) between the normal tissue-derived sample (N) and the cancerous tissue-derived sample (T). In Fig. 1(b), the vertical axis represents signal intensity.
Fig. 1(c) shows the result of a comparison of the quantity of the protein determined by digitizing a signal detected in the quantitative analysis by the NBS method described in the above (5) between the normal tissue-derived sample (N) and the cancerous tissue-derived sample (T). In Fig. 1(c), the vertical axis represents signal intensity.

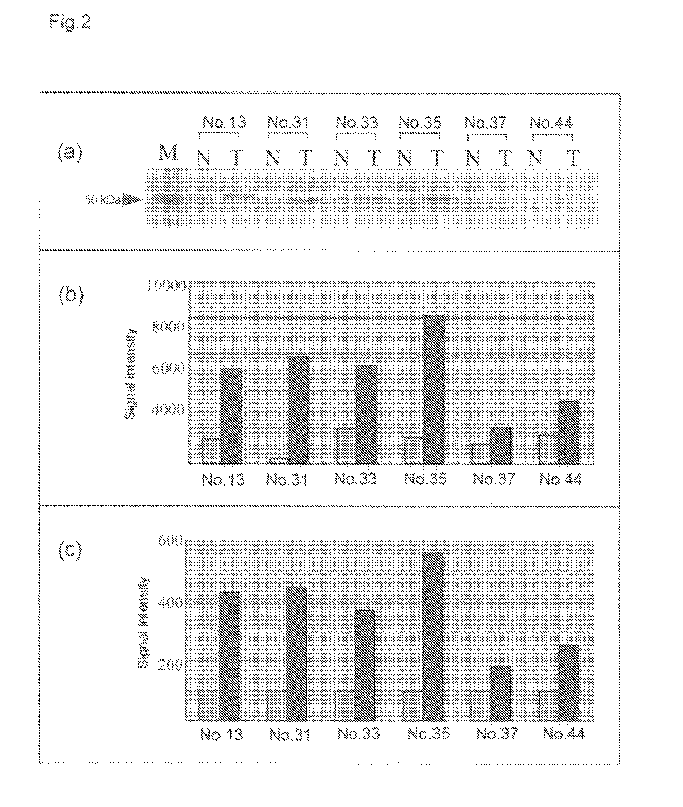

Fig. 2 shows the results of analysis of CNDP dipeptidase 2 as a candidate protein.
Fig. 2(a) shows, in the same manner as in Fig. 1(a), an electrophoretogram obtained by western blotting.
Fig. 2(b) shows, in the same manner as in Fig. 1(b), the result of a comparison of the quantity of the protein determined by digitizing a signal detested in Fig. 2(a) between the normal tissue-derived sample (N) and the cancerous tissue-derived sample (T).
Fig. 2(c) shows, in the same manner as in Fig. 1 (c), the result of a comparison of the quantity of the protein determined by digitizing a signal detected in the quantitative analysis by the NBS method described in the above (5) between the normal tissue-derived sample (N) and the cancerous tissue-derived sample (T).

As shown in Figs. 1 and 2, the candidate proteins, galectin 1 and CNDP dipeptidase 2 showing an upregulation in a cancerous tissue compared to a normal tissue in the quantitative analysis by the NBS method, actually showed such result also in the quantitative analysis by western blotting. Although not shown in the figs, it was actually confirmed that the candidate proteins other than galectin 1 and CNDP dipeptidase 2, that is, reticulocalbin 1, aldolase A, Enolase I, Glucosidase 2 beta subunit, and alpha-crystallin chain B also showed an upregulation in a cancerous tissue compares in a normal tissue.

As described above, there is a good correlation between the result of the quantitative analysis by the NBS method and the result of the quantitative analysis by western blotting.

Therefore, it can be easily estimated that the candidate proteins showing an upregulation in a normal tissue compared to a cancerous tissue in the quantitative analysis by the NBS method will actually show such results also in the quantitative analysis by western blotting.

As described above, the proteins claimed in claim 1 are upregulated in a cancerous tissue comparer to a normal tissue, and the proteins claimed in claim 3 are downregulated in a cancerous tissue compared to a normal tissue. As shown by the analysis using the NBS method, it is apparent that these proteins can be used as tumor markers for renal cancer.

## Claims

1. A tumor marker for renal cancer including at least one protein selected from the group consisting of galectin 1, CNDP dipeptidase 2, reticulocalbin 1, skeletal muscle LIM-protein FHL 1, aldolase A, Enolase I, putative MAPK activating protein (GTRAP3-18), plectin, ribonucleoprotein A2B1, Glucosidase 2 beta subunit, and alpha-crystallin B.

2. A tumor marker for renal cancer including a protein selected from the group consisting of uromodulin, Aminoacylase 1, aldolase B, heparan sulfate proteoglycan, Aquaportin 1, Lipocalin 7, and Raf kinase inhibitor protein.

3. A method for identifying the morbidity of renal cancer by using, as a tumor marker for renal cancer, at least one protein selected from the group consisting of galectin 1, CNDP dipeptidase 2, reticulocalbin 1, skeletal mascle LIM-protein FHL 1, aldolase A, Enolase I, putative MAPK activating protein (GTRAP3-18), plectin, ribonuclesprotein A2B1, Glucosidase 2 beta subunit, and alpha-crystailin B.

4. The method for identifying the morbidity of renal cancer according to claim 3, comprising measuring the level of the protein in a sample derived from a person of interest who is to be examined to identify the morbidity of renal cancer; and comparing an obtained measured level to a normal level of the protein, wherein an upregulation of the obtained measured levee compared to the normal level is used as one of indexes indicating that there is a high possibility that the person of interest has renal cancer.

5. The method for identifying the morbidity of renal cancer according to claim 4, wherein the sample is blood serum or urine and wherein the level of the protein is measured by an examination based on biospecific affinity.

6. A method for identifying the morbidity of renal cancer by using, as a tumor marker for renal cancer, a protein selected from the group consisting of uromodulin, Aminoacylase 1, aldolase B, heparan sulfate proteoglycan, Aquaportin 1, Lipocalin 7, and Raf kinase inhibitor protein.

7. The method for identifying the morbidity of renal cancer according to claim 6, comprising measuring the level of the protein in a sample derived from a. person of interest who is to be examined to identify the morbidity of renal cancer; and comparing an obtained measured level to a normal level of the protein, wherein a downlagulation of the obtained measured level compared to the normal level is used as one of indexes indicating that there is a high possibility that the person of interest has renal cancer.

8. The method for identifying the morbidity of renal cancer according to claim 7, wherein the sample is blood serum or urine and wherein the level of the protein is measured by an examination based on biospecific affinity.
